# EUROPEAN PATENT APPLICATION

(11) **EP 2 345 390 A1**
(43) Date of publication of application: **20.07.2011**
(21) Application number: 10380005.8
(22) Date of filing: 14.01.2010
(51) Int. Cl.: A61F 2/34, A61F 2/30

(54) **Procedure for the manufacture and implant of sponge material of governable density**

(71) Applicant: Socinser 21, S.A., 33392 Gijon Asturias (ES)
(72) Inventor: Garcia Pando, Carlos, 33392 Gijon, Asturias (ES); Garcia Garcia, Manuel Antonio, 33392 Gijon, Asturias (ES); Martin Laguna, Santiago, 33392 Gijon, Asturias (ES); Gonzalez Rodriguez, Manuel Angel, 33392 Gijon, Asturias (ES); Iglesias Rodriguez, Alfonso, 33392 Gijon, Asturias (ES)
(74) Representative: Carvajal y Urquijo, Isabel

(57) **Abstract**

Manufacturing procedure of an implant of governable density and implant manufactured according to the present method that has the phases: to define a three-dimensional shape of the geometry of the implant; to define an osseointegration depth from the superficial layer (2) up to an osseointegration depth layer (1); to generate a solid material: establishing a multidirectional interconnection network creating a net of interconnected cavities to define a spongy structure: originating a lattice that constitutes the resistant structure, with a function of thickness variation of the material taking into account that the lattice does not present ridges in the superficial layer (2); defining a function of volumic density.

## Description

### Field of the invention

The present invention refers to a manufacturing procedure of an implant of sponge material of governable density. The implant can be used in the traumatology field, it can be a dental implant or it can be used for other types of applications in surgery.

### Background of the invention

The number of patients that undergo hip joint replacement (arthroplasty of the hip) grows constantly due to the increase in life expectancy of the population, as well as greater expectations to have a good quality of life which makes younger patients undergo these types of operations to ease the pain and recover their mobility. Hip joint t replacement is the only effective treatment for many patients who suffer from high levels of immobility, dramatically improving their living conditions by allowing them regain their mobility. An implant that does not provide a long term stable fixation and needs to be early replaced causes unnecessary suffering to the patient, as well as an additional social cost since it demands a new surgical treatment and subsequent rehabilitation. Therefore, it is very important to develop products that reduce the risk of this happening.

One of the main factors in orthopedic implants is the capacity of said implant to adhere to the bone wherein it is housed. The ideal adherence mechanism is to achieve that the bone cells of the patient grow around the implant. Traditionally, the promotion of adherence has been performed by means of superficial treatments or coatings over the component to be implanted. A habitual proceeding is to use hydroxyapatite coatings, a ceramic material with an osteoconductive bioactive behaviour, which causes a chemical interaction between the implant and the live tissue, resulting in osseointegration. These types of coatings offer good results in improving the adherence, although they still do not provide optimum conditions to favor osseointegration. Currently, bioactive glasses that posses a much higher osseointegration capacity than the hydroxyapatite are being used, but they are very fragile and they cannot be used in orthopedic surgery, being employed mainly in filling applications. Another type of solution, that is still being developed, is the use of tissue engineering materials (bone morphogenetic proteins -BMPs) and plasma rich in growth factors. These types of active coatings entail a much longer and expensive developing process and bio-sanitary validation. On the other hand, metallic materials have been developed with porous structure whose purpose is to facilitate osseointegration of bone implants. The material is given a structure similar to the morphology of the cancellous bone tissue, which will allow the bone to grow intertwined with the metallic structure.

In this type of porous metals, a chemical process that employs elemental tantalum metal and a process of vapor phase deposition are used. These chemical processes are specific to every material: initially for the tantalum, and more recently they have been obtained for titanium alloys. All these chemical processes, however, cannot be generalized to other types of biocompatible materials.

As well as these chemical methods of metallic foams synthesis, metallic foams have been developed by means of physical methods, particularly by laser sintering and electron beam sintering.

The laser sintering and electron beam sintering techniques offer the possibility of manufacturing self supporting structures, according to a predetermined design. An outstanding application of this technique is the manufacture of implants to be used in traumatology constituted by porous structures with interconnected porosity that promote osseointegration between the implant and the bone, when the bone cells colonize the interstices of the metallic structure.

Different types of laser sintered interconnected porous structures are known in the technique for these kinds of applications, although they are all based on the design of a unit cell constituted by a cluster of rods and nodes that is replicated in space to generate a macroscopic structure. In EP 1683593 different geometric designs are displayed for the unit cell such as rhombohedral, diamond structure, among others. Following a similar methodology, in WO 2007/113862 A1 porous structures are protected in which there is formed a mesh with reticulate structure of circular section in which a periodic structure with the shape of interconnected rings is created.

Specific applications of the use of laser sintering technology and electronic beam sintering have been designed to manufacture a prosthetic element. Thus, WO 2008/146141 describes a cotyle component or acetabular component with a superficial structure consisting in each cell having a polygonal geometry of geodesic type, in such a way that each cell has the same dimension in every zone of the surface of the half sphere. WO 2007/048817 A1 describes a stem-like component wherein a porous surface is formed in a region, constituted by a network structure characterized by a material of invariable thickness all along the network structure and an interstitial geometry substantially polyhedral, having the structure a multilayer geometry.

### Description of the invention

The present invention relates to a procedure and to the prosthetic component or implant manufactured by means of said procedure, being the prosthetic component an acetabular component type, constituted by a porous material. The porosity presents a geometry that corresponds to a concept of adaptive design, instead of replicating a unit cell in space. The implant structure is not constituted by a unit cell that is replicated. The structure is not constituted by thickness rods and invariable cross section which join in nodes forming a polyhedral shape.

Instead, the implant manufactured according to the procedure of the present invention comprises a foamy layer whose technical characteristics are defined by means of some functions that establish the values the variables take from some input parameters, or through delimited values between some extremes that limit the range of a variable. Some of these variables are:
- The thickness of the porous layer;
- The minimum diameter of the pore;
- The maximum diameter of the pore;
- The maximum volumic porosity (minimum effective density);
- The minimum volumic porosity (maximum effective density);

The procedure of the invention allows to vary the volumic porosity (or relative density) of the layer, in a controlled, progressive, constant, and simple manner.

This layer can be stretched, wrinkled, folded or deformed as a flexible solid, control over the local density thereof is kept, and transitions between regions with different characteristics are smooth and progressive, keeping stable mechanic characteristics in the transitions without creating fracture zones in them due to tension concentration. This layer is deposited onto a structure of a solid interior layer of the acetabular component, in order to manufacture the piece set as a mono-block piece.

The foam structure obtained by means of this procedure greatly simulates the real structure of the bone, since the implant manufactured in accordance with the procedure has a biomimetic design, emulating the geometry in which the bone cells intertwine, which is an irregular geometry quite different from the kind of structure obtained with other methods by replicating polyhedral unit cells.

The procedure of the invention allows the implant to be manufactured:
in the typical biocompatible metals: titanium, titanium alloys, stainless steel, tantalum, niobium and cobalt-chromium alloys.
- by means of selective laser sintering, selective laser melting and electron beam melting.

A first aspect of the present invention relates to a procedure for manufacturing an implant of governable density such as the one defined in the appended claims. A second aspect of the present invention relates to an implant such as the one defined in the appended claims.

### Brief description of the drawings

The following is a brief description of a series of drawings which will help understand better the invention relating to an embodiment of said invention, which is presented as a non-limiting example thereof.

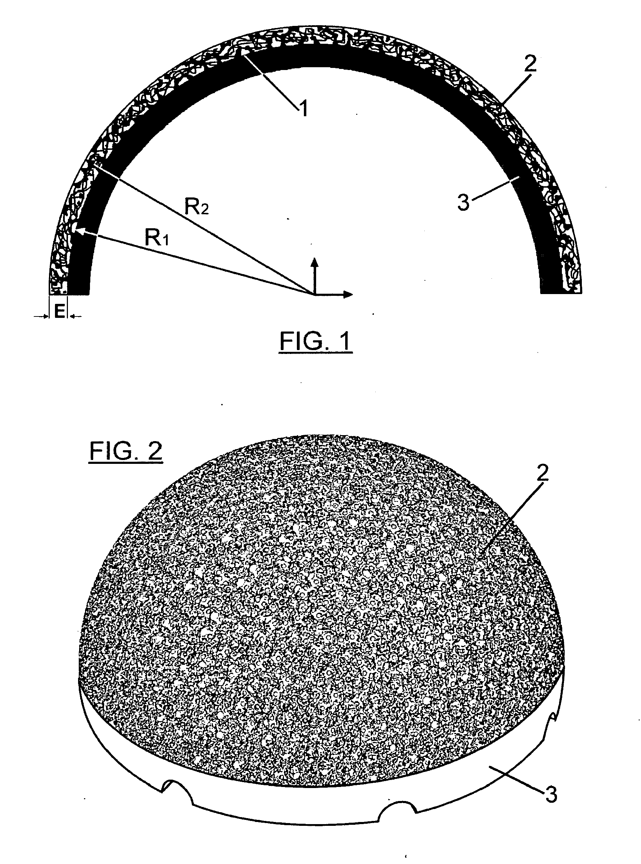

Figure 1 is a section view of an implant manufactured in accordance with the procedure of the present invention.
Figure 2 is a perspective view of a foamy layer of the present invention adapted to a hemispheric shape.

### Description of a preferred embodiment of the invention

There has been developed a method which allows creating a material with a geometry that corresponds to a concept of adaptive design, instead of to a replication of a unit cell in space. The structure is not constituted by a unit cell that is replicated, neither by thickness rods and invariable cross section which join in nodes forming a polyhedral shape.

The foam structure obtained by means of this procedure greatly simulates the real structure of the bone, since the procedure and the implant take into account a biomimetic design, emulating the geometry in which the bone cells intertwine, which is an irregular geometry quite different from the kind of structure obtained with other methods by means of polyhedral unit cells replication.

The very same material composition that constitutes the implant is adapted to the geometry of the piece.

The procedure of the invention allows creating a spongy structure adapted to the ultimate geometry of the piece. Hence, what is achieved is that the foam structure has homogeneity all along its surface and in its thickness.

Thus, an embodiment of the invention relates to a procedure for manufacturing an implant of governable density (by "governable" it is understood that the density is defined by a function that makes the density adopt the established values in the procedure) characterized in that it comprises the following stages:
1a) to define a three-dimensional shape of the geometry of the implant that comprises:
   1a1) delimiting a surface in an implant contour conjugated from a surface where the implant will be housed to generate the perimeter of the implant in a superficial layer (2);
   1a2) specifying a resistant structure configured to bear mechanic loads to which the implant will be subjected;
1b) to define an osseointegration depth from the superficial layer (2) up to an osseointegration depth layer (1):
   1b1) determining a pore size, wherein the size of the pore is determined by the diameter of the cavity that constitutes the pore, being the diameter of the pore, dpore, between 0,3mm ≤dpore ≤1,2mm;
   1b2) determining a density of the pore, wherein the density of the pore is determined by the number of pores by volume unit; when the volumic porosity is maximum, the effective density is minimum, and when the volumic porosity is minimum, the effective density is maximum;
   1b3) determining a material thickness, e, being the thickness between 0,3mm ≤e ≤ 2,5mm;
1c) to generate a solid material:
   1c1) establishing a multidirectional interconnection network creating a net of interconnected cavities to define a spongy structure:
      1c11) without alignment between the cavities;
      1c12) without following predetermined patterns of spatial distribution of the cavities;
      1c13) without forming structures of crystalline type;
      1c14) without forming structures of replicative type;
   1c2) originating a lattice that constitutes the resistant structure, wherein a function of thickness variation of the material takes into account that the lattice does not present ridges in, at least, the superficial layer (2);
   1 c3) defining a function of volumic density:
      1c31 ) depending on, at least, the size of the pore, the density of the pore and the thickness of the material;
      1c32) establishing that in a section from a superficial layer (2) up to an osseointegration depth layer (1) there is a density distribution that rises from a value between 10-30% on the superficial layer (2) up to a value between 70-90% in the osseointegration depth layer (1), 100% corresponding to a solid material;
      1c33) establishing that the density function presents a continuous, limited, without peculiarities behaviour per layers, to guarantee uniform density values along a layer.

According to other characteristics of the invention:
2. The procedure further comprises:
   2a) adding a configured substrate to favour a culture and/or tissue growth and thus improve the integration of the implant in the tissue:
      2a1) according to a distribution map that comprises:
         2a11) a function of superficial concentration on each layer of the implant;
         2a12) a function of in-depth concentration on different layers of the implant; The function of superficial concentration on each layer of the implant and the function of in-depth concentration on different layers of the implant can be a continuous and gradual progression from one kind of concentration to another so as to avoid interface effects.
3. The substrate is added in the osseointegration depth.
4. The phase 1c) comprises an operation selected among: selective laser sintering, selective laser melting and electron beam melting.
5. The implant is manufactured in accordance with the procedure described above, from a sponge material of governable density, comprising said implant:
   5a) a three-dimensional shape of the geometry of the implant that comprises:
      5a1) a surface in an implant contour conjugated from a surface where the implant will be housed to generate the perimeter of the implant in a superficial layer (2);
      5a2) a resistant structure configured to bear mechanic loads to which the implant will be subjected;
   5b) an osseointegration depth E from the superficial layer (2) up to an osseointegration depth layer (1) that comprises:
      5b1) a plurality of pores, wherein a size of the pore is determined by the diameter of the cavity that constitutes the pore, being the diameter of the pore, dpore, between 0,3mm ≤dpore ≤1,2mm;
      5b2) a density of the pore, wherein the density of the pore is determined by the number of pores by volume unit;
      5b3) a material thickness, e, between 0,3mm ≤e ≤2,5mm;
   5c) a solid material that comprises:
      5c1) a multidirectional interconnection network that consists in a net of interconnected cavities to define a spongy structure:
         5c11) without aligned cavities;
         5c12) without predetermined patterns of spatial distribution of the cavities;
         5c13) without structures of crystalline type;
         5c14) without structures of replicative type;
      5c2) a resistant structure in the form of a lattice, wherein a function of thickness variation of the material is configured in such a way that the lattice does not present ridges in, at least, the superficial layer (2);
      5c3) a function of volumic density:
         5c31) that depends on, at least, the size of the pore, the density of the pore and the thickness of the material;
         5c32) that in a section from a superficial layer (2) up to an osseointegration depth layer (1) there is a density distribution that rises from a value between 10-30% on the superficial layer (2) up to a value between 70-90% in the osseointegration depth layer (1), 100% corresponding to a solid material;
         5c33) that has a density distribution per layers that is continuous, limited, without peculiarities or local extremes to guarantee uniformity.
6. The three-dimensional shape is a hemispheric cortex that consists of:
   6a) an interior radius R1 that defines the osseointegration depth layer (1);
   6b) an exterior radius R2 that defines the superficial layer (2);
   6c) an osseointegration depth E comprised between the superficial layer (2) and the osseointegration depth layer (1), being the osseointegration depth E between 2 ≤E ≤ 4,5mm.
7. The three-dimensional shape of the piece provides anisotropy to the implant to optimize a mechanic resistance/weight relation of the implant.
8. The implant further comprises an extra solid layer (3) joint to the osseointegration depth layer (1).
9. The implant is a material selected among biocompatible materials.
10. The material is selected among: titanium, titanium alloys, stainless steel, tantalum, niobium and cobalt-chromium alloys.

## Claims

1. Procedure for the manufacture of an implant of governable density **characterized in that** it comprises the following stages:
1 a) to define a three-dimensional shape of the geometry of the implant that comprises:
1a1) delimiting a surface in an implant contour conjugated from a surface where the implant will be housed to generate the perimeter of the implant in a superficial layer (2);
1a2) specifying a resistant structure configured to bear mechanic loads to which the implant will be subjected;
1b) to define an osseointegration depth from the superficial layer (2) up to an osseointegration depth layer (1):
1b1) determining a pore size, wherein the size of the pore is determined by the diameter of the cavity that constitutes the pore, being the diameter of the pore, dpore, between 0,3mm ≤dpore ≤1,2mm;
1b2) determining a density of the pore, wherein the density of the pore is determined by the number of pores by volume unit;
1b3) determining a material thickness, e, being the thickness between 0,3mm ≤e ≤2,5mm;
1c) to generate a solid material:
1c1) establishing a multidirectional interconnection network creating a net of interconnected cavities to define a spongy structure:
1 c11) without existing alignment between the cavities;
1c12) without following predetermined patterns of spatial distribution of the cavities;
1c13) without forming structures of crystalline type;
1c14) without forming structures of replicative type;
1c2) originating a lattice that constitutes the resistant structure, wherein a function of thickness variation of the material takes into account that the lattice does not present ridges in, at least, the superficial layer (2);
1 c3) defining a function of volumic density:
1c31 ) depending on, at least, the size of the pore, the density of the pore and the thickness of the material;
1c32) establishing that in a section from a superficial layer (2) up to an osseointegration depth layer (1) there is a density distribution that rises from a value between 10-30% on the superficial layer (2) up to a value between 70-90% in the osseointegration depth layer (1), 100% corresponding to a solid material;
1c33) establishing that the density function presents a continuous, limited, without peculiarities behaviour per layers, to guarantee uniform density values along a layer.

2. Procedure according to claim 1, **characterized in that** it further comprises:
2a) adding a configured substrate to favour a culture and/or tissue growth and thus improve the integration of the implant in the tissue:
2a1) according to a distribution map that comprises:
2a11) a function of superficial concentration on each layer of the implant;
2a12) a function of in-depth concentration on different layers of the implant;

3. Procedure according to claim 2, **wherein** the substrate is added in the osseointegration depth.

4. Procedure according to any of the preceding claims 1-3, **characterized in that** the stage 1c) consists of an operation selected among: selective laser sintering, selective laser melting and electron beam melting.

5. Implant **characterized in that** it is manufactured in accordance with the procedure of the preceding claims 1-4 from a sponge material of governable density, said implant comprising:
5a) a three-dimensional shape of the geometry of the implant that comprises:
5a1) a surface in an implant contour conjugated from a surface where the implant will be housed to generate the perimeter of the implant in a superficial layer (2);
5a2) a resistant structure configured to bear mechanic loads to which the implant will be subjected;
5b) an osseointegration depth E from the superficial layer (2) up to an osseointegration depth layer (1) that comprises:
5b1) a plurality of pores, wherein a size of the pore is determined by the diameter of the cavity that constitutes the pore, being the diameter of the pore, dpore, between 0,3mm ≤dpore ≤1,2mm;
5b2) a density of the pore, wherein the density of the pore is determined by the number of pores by volume unit;
5b3) a material thickness, e, between 0,3mm ≤e ≤2,5mm;
5c) a solid material that comprises:
5c1) a multidirectional interconnection network that consists in a net of interconnected cavities to define a spongy structure:
5c11) without aligned cavities;
5c12) without predetermined patterns of spatial distribution of the cavities;
5c13) without structures of crystalline type;
5c14) without structures of replicative type;
5c2) a resistant structure in the form of a lattice, wherein a function of thickness variation of the material is configured in such a way that the lattice does not present ridges in, at least, the superficial layer (2);
5c3) a function of volumic density:
5c31) that depends on, at least, the size of the pore, the density of the pore and the thickness of the material;
5c32) that in a section from a superficial layer (2) up to an osseointegration depth layer (1) there is a density distribution that rises from a value between 10-30% on the superficial layer (2) up to a value between 70-90% in the osseointegration depth layer (1), 100% corresponding to a solid material;
5c33) that has a density distribution per layers that is continuous, limited, without peculiarities or local extremes to guarantee uniformity.

6. The implant according to claim 5, **wherein** the three-dimensional shape is a hemispheric cortex that consists of:
6a) an interior radius R1 that defines the osseointegration depth layer (1);
6b) an exterior radius R2 that defines the superficial layer (2);
6c) an osseointegration depth E comprised between the superficial layer (2) and the osseointegration depth layer (1), being the osseointegration depth E between 2 ≤E ≤ 4,5mm.

7. The implant according to any of the preceding claims 5-6, **wherein** the three-dimensional shape of the piece provides anisotropy to the implant to optimize an implant mechanic resistance/weight relation.

8. The implant according to any of the preceding claims 5-7 **characterized in tha**t it further comprises an extra solid layer (3) joint to the osseointegration depth layer (1).

9. The implant according to any of the preceding claims 5-8 **characterized in that** it is a material selected among biocompatible materials.

10. The implant according to claim 9 **wherein** the material is selected between: titanium, titanium alloys, stainless steel, tantalum, niobium and cobalt-chromium alloys.
